# FASCICULE DE BREVET EUROPEEN

(11) **EP 1 003 844 B1**
(45) Date de publication et mention de la délivrance du brevet: **22.02.2006**
(21) Numéro de dépôt: 98942723.2
(22) Date de dépôt: 11.08.1998
(51) Int. Cl.: C12N 9/12, C12N 15/54, C12N 15/81

(54) **KINASE ACTIVATRICE DES PROTEINES-KINASES CYCLINE DEPENDANTES, ET SES UTILISATIONS**
KINASE DIE CYCLIN-ABHÄNGIGEN PROTEINKINASEN AKTIVIERT, UND DEREN VERWENDUNGEN
KINASE ACTIVATING DEPENDENT CYCLIN PROTEIN KINASES, AND THEIR USES

(30) Priorité: 12.08.1997 FR 9710287
(43) Date de publication de la demande: 31.05.2000
(73) Titulaire: COMMISSARIAT A L'ENERGIE ATOMIQUE, 75752 Paris Cédex 15 (FR); Institut Curie, 75248 Paris Cedex 05 (FR); CENTRE NATIONAL DE LA RECHERCHE SCIENTIFIQUE (CNRS), 75794 Paris Cedex 16 (FR)
(72) Inventeur: FAYE, Gérard, F-94110 Arcueil (FR); VALAY, Jean, Gabriel, F-38190 Bernin Cedex 22 (FR); MANN, Carl, F-78114 Magny les Hameaux (FR); THURET, Jean-Yves, F-91400 Orsay (FR)
(74) Mandataire: Vialle-Presles, Marie José
(86) Numéro de dépôt international: PCT/FR1998/001788
(87) Numéro de publication internationale: WO 1999/007836

(56) Documents cités:
- WO-A-97/16447
- THURET J.Y. ET AL.: "Civ1 (CAK in vivo), a novel Cdk-activating kinase" CELL, vol. 86, no. 4, 23 août 1996, pages 565-576, XP002065325 cité dans la demande
- HERMAN ET AL: 'A novel protein kinase ...' CELL vol. 64, 1991, pages 425 - 437
- SHERLOCK ET AL: 'Molecular cloning and analysis of CDC28 and cyclin homologues from the human fungal pathogen Candida albicans' MOL.GEN.GENET. vol. 245, 1994, pages 716 - 723

### LISTAGE DE SEQUENCE

<110> FAYE, Gérard
   VALAY, Jean Gabriel
   MANN, Carl
   THURET, Jean-Yves
   CEA
   INSTITUT CURIE
   CNRS
<120> KINASE ACTIVATRICE DES PROTEINE-KINASES CYCLINE DEPENDANTES, ET SES UTILISATIONS
<130> MJPrm263/33
<140>
   <141>
<150> FR 9710287
   <151> 1997-08-12
<160> 2
<170> PatentIn Vers. 2.0
<210> 1
   <211> 1020
   <212> ADN
   <213> Candida albicans
<220>
   <221> CDS
   <222> (1)..(1020) 
<400> 1
atg aag ttg tca gat tat tat ata gac aag gaa tta att tac aat agt     48
Met Lys Leu Ser Asp Tyr Tyr Ile Asp Lys Glu Leu Ile Tyr Asn Ser
1               5                   10                  15

gcc att tct gat ata tat acg gct att gat aag ttt aat aac tta cca     96
Ala Ile Ser Asp Ile Tyr Thr Ala Ile Asp Lys Phe Asn Asn Leu Pro
            20                  25                  30

gta tgt ctt aaa ata gtt gat gaa gat ttc agt ctt cca cca cat tca     144
Val Cys Leu Lys Ile Val Asp Glu Asp Phe Ser Leu Pro Pro His Ser
        35                  40                  45

atc cat cga gaa gtt ctt ata ctt aaa act ttg aaa cca cat cca aac     192
Ile His Arg Glu Val Leu Ile Leu Lys Thr Leu Lys Pro His Pro Asn
    50                  55                  60

ata att gaa tat ttt aat gat ctt aaa att tgt gac gat att ata tta     240
Ile Ile Glu Tyr Phe Asn Asp Leu Lys Ile Cys Asp Asp Ile Ile Leu
65                  70                  75                  80

gtc acc aaa ttg tat cgt tat gat ttg agt caa ttg att gaa att aca     288
Val Thr Lys Leu Tyr Arg Tyr Asp Leu Ser Gln Leu Ile Glu Ile Thr
                85                  90                  95

aaa tat tgt aaa cga aca aca cga ttt att tat ggt att aat ggt aat     336
Lys Tyr Cys Lys Arg Thr Thr Arg Phe Ile Tyr Gly Ile Asn Gly Asn
            100                 105                 110

ctt gtt agt aat caa tat aca ctt gct aat gaa att gaa gaa aaa gat     384
Leu Val Ser Asn Gln Tyr Thr Leu Ala Asn Glu Ile Glu Glu Lys Asp
        115                 120                 125
 
atc aaa tta tgg tta aaa tca atg agt tca gga ctt gaa ttt att cat 432
Ile Lys Leu Trp Leu Lys Ser Met Ser Ser Gly Leu Glu Phe Ile His
    130                 135                 140

tca caa ggg ata att cat cgt gat ata aaa ccc agt aat att ttc ttt 480
Ser Gln Gly Ile Ile His Arg Asp Ile Lys Pro Ser Asn Ile Phe Phe
145                 150                 155                 160

gcc cgg gat gat ata aca caa ccg att att gga gat ttt gat att tgt 528
Ala Arg Asp Asp Ile Thr Gln Pro Ile Ile Gly Asp Phe Asp Ile Cys
                165                 170                 175

tat gat tta aaa ctg cca cct aaa gat gaa ccc cct atg gcg aaa tat 576
Tyr Asp Leu Lys Leu Pro Pro Lys Asp Glu Pro Pro Met Ala Lys Tyr
            180                 185                 190

att gat gta tct aca ggt att tat aaa gca cca gaa ttg att ctt ggt 624
Ile Asp Val Ser Thr Gly Ile Tyr Lys Ala Pro Glu Leu Ile Leu Gly
        195                 200                 205

ata act aat tat gaa tat gaa att gat att tgg tca ttg ggt ata att 672
Ile Thr Asn Tyr Glu Tyr Glu Ile Asp Ile Trp Ser Leu Gly Ile Ile
    210                 215                 220

ttg act ggt tta tat tca gaa aat ttt caa agt gtt tta gtc aaa gat 720
Leu Thr Gly Leu Tyr Ser Glu Asn Phe Gln Ser Val Leu Val Lys Asp
225                 230                 235                 240

gat aaa gaa ttg act aat gat tct cat gtt agt gat tta tat tta tta 768
Asp Lys Glu Leu Thr Asn Asp Ser His Val Ser Asp Leu Tyr Leu Leu
                245                 250                 255

aat caa ata ttt gaa aat ttc ggt aca ccc aat tta act gat ttt gaa 816
Asn Gln Ile Phe Glu Asn Phe Gly Thr Pro Asn Leu Thr Asp Phe Glu
            260                 265                 270

gat gaa tta ttt tgt gat gaa tat aat aat gaa aac ttg cat ttt aaa 864
Asp Glu Leu Phe Cys Asp Glu Tyr Asn Asn Glu Asn Leu His Phe Lys
        275                 280                 285

aaa ttc aat tta caa aaa tat cct aga aaa gat tgg gat att att tta 912
Lys Phe Asn Leu Gln Lys Tyr Pro Arg Lys Asp Trp Asp Ile Ile Leu
    290                 295                 300

cct cga tgc aat gat gat ttc atg aaa gaa att ttt acc aag atg att 960
Pro Arg Cys Asn Asp Asp Phe Met Lys Glu Ile Phe Thr Lys Met Ile
305                 310                 315                 320

aga tat gat cga agt aaa aga ata act tct aaa gaa atc tta caa tta 1008
Arg Tyr Asp Arg Ser Lys Arg Ile Thr Ser Lys Glu Ile Leu Gln Leu
                325                 330                 335

atg tta gat tga 1020
Met Leu Asp
340
<210> 2
   <211> 339
   <212> PRT
   <213> Candida albicans

<400> 2
Met Lys Leu Ser Asp Tyr Tyr Ile Asp Lys Glu Leu Ile Tyr Asn Ser
1               5                   10                  15

Ala Ile Ser Asp Ile Tyr Thr Ala Ile Asp Lys Phe Asn Asn Leu Pro
            20                  25                  30

Val Cys Leu Lys Ile Val Asp Glu Asp Phe Ser Leu Pro Pro His Ser
        35                  40                  45

Ile His Arg Glu Val Leu Ile Leu Lys Thr Leu Lys Pro His Pro Asn
    50                  55                  60

Ile Ile Glu Tyr Phe Asn Asp Leu Lys Ile Cys Asp Asp Ile Ile Leu
65                  70                  75                  80

Val Thr Lys Leu Tyr Arg Tyr Asp Leu Ser Gln Leu Ile Glu Ile Thr
                85                  90                  95

Lys Tyr Cys Lys Arg Thr Thr Arg Phe Ile Tyr Gly Ile Asn Gly Asn
            100                 105                 110

Leu Val Ser Asn Gln Tyr Thr Leu Ala Asn Glu Ile Glu Glu Lys Asp
        115                 120                 125

Ile Lys Leu Trp Leu Lys Ser Met Ser Ser Gly Leu Glu Phe Ile His
    130                 135                 140

Ser Gln Gly Ile Ile His Arg Asp Ile Lys Pro Ser Asn Ile Phe Phe
145                 150                 155                 160

Ala Arg Asp Asp Ile Thr Gln Pro Ile Ile Gly Asp Phe Asp Ile Cys
                165                 170                 175

Tyr Asp Leu Lys Leu Pro Pro Lys Asp Glu Pro Pro Met Ala Lys Tyr
            180                 185                 190

Ile Asp Val Ser Thr Gly Ile Tyr Lys Ala Pro Glu Leu Ile Leu Gly
        195                 200                 205

Ile Thr Asn Tyr Glu Tyr Glu Ile Asp Ile Trp Ser Leu Gly Ile Ile
    210                 215                 220

Leu Thr Gly Leu Tyr Ser Glu Asn Phe Gln Ser Val Leu Val Lys Asp
225                 230                 235                 240

Asp Lys Glu Leu Thr Asn Asp Ser His Val Ser Asp Leu Tyr Leu Leu
                245                 250                 255

Asn Gln Ile Phe Glu Asn Phe Gly Thr Pro Asn Leu Thr Asp Phe Glu
            260                 265                 270

Asp Glu Leu Phe Cys Asp Glu Tyr Asn Asn Glu Asn Leu His Phe Lys
        275                 280                 285

Lys Phe Asn Leu Gln Lys Tyr Pro Arg Lys Asp Trp Asp Ile Ile Leu
    290                 295                 300

Pro Arg Cys Asn Asp Asp Phe Met Lys Glu Ile Phe Thr Lys Met Ile
305                 310                 315                 320

Arg Tyr Asp Arg Ser Lys Arg Ile Thr Ser Lys Glu Ile Leu Gln Leu
                325                 330                 335

Met Leu Asp

## Description

La présente Invention est relative à une nouvelle kinase de *Candida albicans,* activatrice des protéine-kinases cycline dépendantes, et à ses utilisations.

Les protéine-kinases cycline dépendantes (Cdk) sont des régulateurs du cycle de division cellulaire chez les eucaryotes, essentiels aussi bien au niveau de la transition Gl/S que de la transition G2/M du cycle cellulaire. La CDC28 de *Saccharomyces cerevisae* et la CDC2 de *Schizosaccharomyces pombe* sont les premières Cdk qui ont été identifiées.

L'activation des Cdk nécessite à la fois la fixation d'une molécule de cycline, et la phosphorylation de la Cdk sur un résidu thréonine conservé, situé dans une région appelée : « boucle T ».

Il a été montré que cette phosphorylation est effectuée par une kinase dénommée : « kinase activatrice des Cdk » (CAK), qui, chez les vertébrés, se présente sous forme d'un hétérotrimère comprenant une sous-unité catalytique dénommée Cdk7, une sous-unité de type cycline, dénommée cycline H, et un facteur MAT-1 [pour revue, cf. SOLOMON, Trends Biochem. Sci. 19, 496-500 (1994)]. Le complexe Cdk7-cycline H est en outre un composant du complexe TFIIH, nécessaire à la transcription basale des gènes par l'ARN polymérase II, et intervient dans la phosphorylation des séquences répétées du domaine carboxy-terminal (CTD) de la grande sous-unité de cette polymérase.

Chez la levure scissipare *Schizosaccharomyces pombe*, un complexe similaire à Cdk7-cycline H, comprenant une sous-unité catalytique dénommée Crk1, et une cycline régulatrice dénommée Mcs2 a été identifié. Il a été montré que le gène *Crk1* était essentiel pour la viabilité cellulaire, et il a été observé *in vitro* que le complexe Crk1-Mcs2 était associé à l'activité CAK et à l'activité CTD-kinase [BUCK et al., EMBO J., 14(24), 6173-83 (1995) ; DAMAGNEZ et al., EMBO J., 14(24), 6164-72, (1995)].

Chez la levure bourgeonnante *Saccharomyces cerevisiae,* un complexe comprenant une kinase (Kin28) et une cycline (Ccl1) respectivement apparentées, au niveau de leur séquence, aux kinases Cdk7 et Crk1, et aux protéines régulatrices cycline H et Mcs2 a également été identifié. Le complexe Kin28-Ccl1 fait partie du complexe TFIIH et possède une activité CTD-kinase, mais n'intervient pas dans l'activité CAK.

Récemment, les inventeurs ont identifié une kinase responsable de l'activité CAK chez *Saccharomyces cerevisiae.* Cette kinase a été dénommée CIV1 (CAK in *vivo),* et le gène correspondant a été dénommé *CIV1* (THURET et al., Cell, 86(4), 1996). Ces résultats ont été confirmés par d'autres équipes [KALDIS et al., Cell, 86(4), 553-564 (1996) ; ESPINOZA et al., Science, 273 (5282), 1714-1717 (1996)]. La CAK de *Saccharomyces cerevisiae* est globalement apparentée à la famille des sérine-thréonine-kinases, et en particulier aux protéine-kinases CDC2 et CDC28, et se différencie des CAK précédemment identifiées chez d'autres organismes par l'absence du motif conservé riche en glycines GxGx (Y/F) GxV, qui est présent dans la plupart des protéine-kinases, la présence d'insertions de 5 à 29 acides aminés, situées entre les éléments de structure secondaire conservés dans la famille des Cdk, et par le fait que son activité CAK ne nécessite pas son incorporation dans un complexe enzymatique.

L'activité CAK de CIV1 étant essentielle pour la survie et la division cellulaire, les Inventeurs ont entrepris de rechercher s'il existait, chez des levures pathogènes, des gènes homologues à CIV1, codant pour des protéine-kinases possédant une activité CAK. En effet, dans ce cas, l'obtention de moyens de régulation de cette activité, et en particulier d'inhibiteurs, présenterait un grand intérêt sur le plan industriel ou thérapeutique, principalement pour l'obtention de fongicides.

Dans ce but, les Inventeurs ont d'abord entrepris le criblage de banques d'ADN de la levure pathogène *Candida albicans*, en utilisant des sondes dérivées des différentes régions du gène *CIV1* de *Saccharomyces cerevisiae.* Cependant, aucune des sondes utilisées n'a permis de détecter la présence de séquences homologues dans le génome de Candida *albicans.*

Les Inventeurs ont toutefois recherché si *Candida albicans* possédait éventuellement un analogue fonctionnel de la CAK de *Saccharomyces cerevisiae*, en cherchant s'il existait chez *Candida albicans* un ou des gènes capables de restaurer chez *Saccharomyces cerevisiae* la fonction CAK dans un mutant thermosensible du gène *CIV1.* Ils sont ainsi parvenus à identifier un gène de *Candida albicans* capable de complémenter à lui seul la fonction CAK déficiente du mutant.

La séquence de ce gène, dénommé *CaCIV1,* a été déterminée ; elle est représentée dans la liste de séquences en annexe sous le numéro SEQ ID NO:1 ; la séquence de son produit de traduction, dénommé CaCIV1, est représentée sous le numéro SEQ ID NO:2.

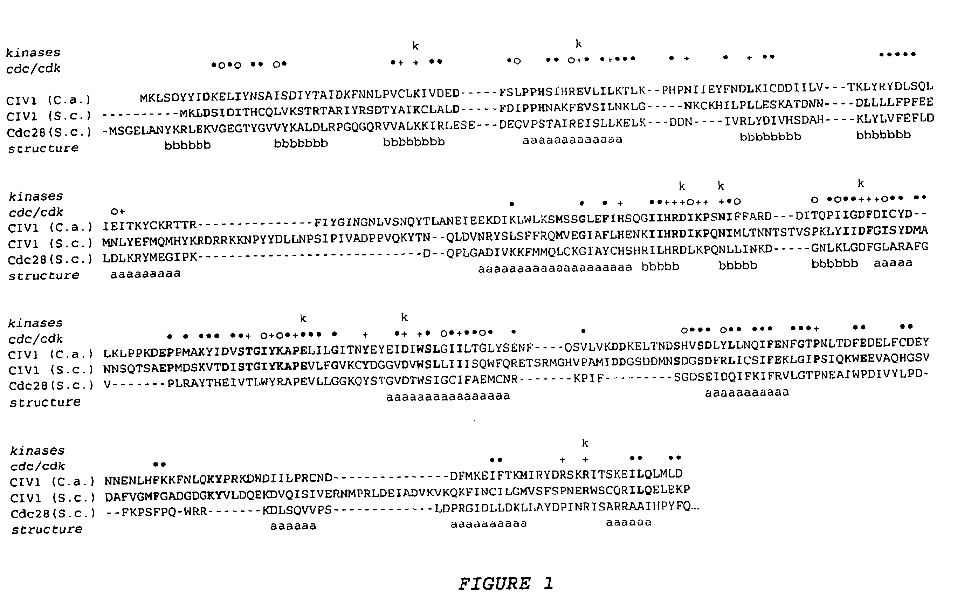

La figure 1 représente la comparaison de la séquence d'acides aminés (code 1-lettre) de CaCIV1, avec celle de la CAK de *Saccharomyces cerevisiae* (dénommée ScCIV1), et avec celle de la kinase CDC28 de *Saccharomyces cerevisiae* (dénommée ScCDC28). Les résidus conservés chez ScCIV1 et CaCIV1 sont en caractères gras.

Légende des annotations de la figure 1 :
k = résidu conservé dans la plupart des protéine-kinases ;
• = résidu souvent présent dans la famille des Cdk ;
o = résidu toujours présent dans la famille des Cdk ;
+ = résidu présent dans la famille des Cdk et dans ScCIV1 ;

Structures secondaires : a = hélice □ ; b = feuillet β.

CaCIV1 ne présente au niveau de la séquence globale en acides aminés, qu'une identité de 28% avec la CAK de *Saccharomyces cerevisiae*, ScCIV1.

Cependant, les similitudes constatées entre ScCIV1 et CaCIV1 permettent de définir une famille de kinases, dénommée ci-après CIV1, regroupant des protéines possédant les caractéristiques suivantes :
- elles sont dépourvues du motif GxGx(Y/F)GxV, dans lequel G représente la glycine, x représente un acide aminé quelconque, Y/F représente soit la tyrosine soit la phénylalanine, V représente la valine.
- elles possèdent une activité CAK non-cycline dépendante.

La présente invention a pour objet une protéine-kinase de *Candida albicans* appartenant à la famille CIV1 telle que définie ci-dessus à l'exception de la CAK ScCIV1 de Saccharomyces cerevisae.

Une protéine-kinase conforme à l'invention est représentée dans la liste de séquences en annexe sous le numéro SEQ ID NO:2.

La présente Invention a également pour objet une séquence d'acide nucléique codant pour une protéine-kinase conforme à l'invention.

Une séquence d'acide nucléique conforme à l'invention est par exemple constituée par la séquence SEQ ID NO:1 de la liste de séquences en annexe.

Une séquence d'acide nucléique conforme à l'invention peut être isolée et clonée à partir de *Candida albicans,* en utilisant comme sondes d'hybridation, et/ou amorces d'amplification, des fragments d'acide nucléique d'au moins 18 pb du polynucléotide SEQ ID NO:1, codant pour une séquence peptidique spécifique de la CAK SEQ ID NO:2, ou leurs complémentaires.

La présente invention a également pour objet tout vecteur recombinant, et en particulier tout vecteur d'expression, résultant de l'insertion d'au moins une séquence d'acide nucléique conforme à l'invention dans un vecteur approprié. Le choix d'un vecteur approprié peut être effectué aisément par l'homme du métier, parmi les nombreux vecteurs disponibles, selon la cellule-hôte choisie pour multiplier et/ou exprimer un acide nucléique conforme à l'invention.

L'invention englobe également des cellules procaryotes ou eucaryotes transformées par une séquence d'acide nucléique conforme à l'invention. Ces cellules transformées peuvent être utilisées en particulier pour exprimer une kinase conforme à l'invention, par exemple afin de la purifier à partir des cultures cellulaires, par exemple en utilisant des techniques similaires à celles précédemment décrites par les Inventeurs pour la CIV1 de *Saccharomyces cerevisiae,* (THURET et al., 1996, publication citée ci-dessus), ou bien afin de détecter son activité par un test approprié de viabilité cellulaire.

La mise en évidence par les Inventeurs de l'homologie fonctionnelle des kinases ScCIV1 et CaCIV1 permet d'envisager de nombreuses applications pour cette famille de kinases.

En particulier, du fait qu'il apparaît que l'activité CAK non cycline-dépendante de cette famille de kinases est essentielle pour la survie et la division cellulaire, des substances inhibitrices de cette activité peuvent être utilisables comme fongicides, soit en tant que médicaments, soit sur le plan industriel.

Par exemple, pour cribler des substances fongicides, telles que des substances actives sur *Candida albicans,* on mesure l'activité kinase de CaCIV1, ou de l'un de ses homologues fonctionnels constitué par une CAK non cycline-dépendante de la famille CIV1, en présence de chacun des produits dont on souhaite déterminer les propriétés fongicides, et l'on sélectionne les produits ayant un effet inhibiteur sur cette activité.

On peut effectuer un tel criblage en mesurant l'activité kinase d'une CAK de la famille CIV1, en présence d'activateurs ou d'inhibiteurs potentiels à tester. L'activité kinase peut par exemple être mesurée *in vitro,* soit directement en détectant la phosphorylation d'un peptide ou d'une protéine substrat, par exemple CDC28 ou Cdk2, ou la protéine MBP (myelin basic protein), dans un mélange réactionnel approprié, soit indirectement, en détectant et/ou mesurant l'activité de la protéine substrat lorsque celle-ci dépend de la phosphorylation.

L'activité kinase peut également être mesurée in vivo, par un test de viabilité cellulaire ; par exemple, l'activité kinase de CaCIV1 peut avantageusement être mesurée dans des cellules d'un mutant de *Saccharomyces cerevisiae* n'exprimant pas la CAK ScCIV1, transformées par le gène *CaCIVl.*

L'invention englobe également l'utilisation, d'un produit sélectionné comme indiqué ci-dessus pour ses propriétés inhibitrices d'une CAK non cycline-dépendante de la famille CIV1 pour l'obtention d'un fongicide

La présente invention sera mieux comprise à l'aide du complément de description qui va suivre, qui se réfère à un exemple illustrant la mise en évidence de l'activité CAK de CaCIV1, et le clonage du gène correspondant.

### Exemple :

La souche de *Saccharomyces cerevisiae,* CMY975 (génotype *CIV1-2 ura3 leu2 trp1 lys2 ade2 ade3*) porte une mutation thermosensible du gène *CIV1* et, de ce fait, pousse à 24°C, mais pas à 37°C.

Une culture de cette souche a été transformée par la méthode à l'acétate de lithium [SCHIESTL et GIETZ, Current Genetics, 16, 339-346, (1989)] avec une banque de fragments génomiques Sau3A de *Candida albicans* clonés dans le site BamHI du vecteur YEp24 (multicopie-*URA*3) [Botstein et al., Gene *8*, 17-24, (1979)].

Les cellules CMY975 sont étalées sur des boîtes contenant un milieu synthétique dépouvu d'uracile, et cultivées à 37°C.

Dix colonies de CMY975 poussant dans ces conditions ont été obtenues. Les plasmides YEp24 contenant des inserts de Candida albicans ont été récupérés à partir de chacune ces colonies, et amplifiés chez *Escherichia coli.*

La carte de restriction de chacun de ces plasmides a été établie, et a permis de constater que tous les inserts provenaient d'une même région du génome de Candida *ablicans.* Le séquencage de cette région a permis de mettre en évidence un même cadre ouvert de lecture codant pour une protéine kinase de 339 acides aminés, ce qui montre que les 10 inserts obtenus séparément correspondent à un seul et même gène de Candida *albicans.* Ce gène a été dénommé *CaCIV1.*

La séquence de *CaCIV1* est représentée dans la liste de séquences en annexe sous le numéro SEQ ID NO:1, et celle de la protéine CaCIV1 est représentée sous le numéro SEQ ID NO: 2. La comparaison de la séquence de la protéine CaCIV1 avec les séquences disponibles sur les bases de données fait apparaître que cette protéine possède seulement 28% d'acides aminés identiques avec la CAK de *Saccharomyces cerevisiae* ScCIV1, et 24% d'acides aminés identiques avec les Cdk ScCDC28 de *Saccharomyces cerevisiae* et la CaCDC 28 de Candida *albicans.*

Un fragment génomique BamHI-ClaI de 3 kb contenant le gène *CaCIV1* a été sous-cloné dans le plasmide centromérique TRP1 pRS414 [SIKORSKI et HIETER, Genetics, 122, 19-27 (1989)]. Ce plasmide a été utilisé pour transformer un mutant de *Saccharomyces cerevisiae* dans lequel la séquence *ScCIV1* est délétée du génome et contenant le gène *ScCIV1* sur un plasmide réplicatif portant également le gène de sélection URA3 (souche CMY116 du génome *ura3 leu2 trp1 lys2 civ1 LEU2*/*pJG43* (*URA3-ScCIV1*)*.* Les cellules transformées avec le plasmide pRS414-*CaCIV1* sont viables après la contre-sélection et la perte du plasmide pJG43 (URA3-ScCIV1), ce qui confirme que le gène *CaCIV1* peut fonctionner à la place du gène essentiel *ScCIV1.* Donc, *CaCIV1* code pour un homologue fonctionnel de *ScCIV1,* et suffit à restaurer l'activité CAK.

## Revendications

1. Protéine-kinase possédant une activité CAK non-cycline dépendante, **caractérisée en ce qu'**elle répond à la séquence représentée dans la liste de séquences en annexe sous le numéro SEQ ID NO:2.

2. Polynucléotide codant pour une protéine-kinase selon la revendication 1.

3. Polynucléotide selon la revendication 2, **caractérisé en ce qu'**il répond à la séquence représentée dans la liste de séquences en annexe sous le numéro SEQ ID NO:1.

4. Polynucléotide codant pour une protéine kinase possédant les caractéristiques suivantes :
- elle est dépourvue du motif GxGx (Y/F) GxV, dans lequel G représente la glycine, x représente un acide aminé quelconque, Y/F représente soit la tyrosine soit la phénylalanine, V représente la valine ;
- elle possède une activité CAK non-cycline dépendante ;
ledit polynucléotide étant **caractérisé en ce qu'**il est susceptible d'être isolé à partir de *Candida albicans* à l'aide d'une sonde d'acide nucléique constituée par un fragment d'au moins 18 pb du polynucléotide SED ID NO:1 codant pour une séquence peptidique spécifique d'une protéine-kinase selon la revendication 1, ou par son complémentaire.

5. Protéine-kinase codée par un acide nucléique selon la revendication 4.

6. Vecteur recombinant résultant de l'insertion d'un polynucléotide selon une quelconque des revendications 2 à 4 dans un vecteur approprié.

7. Cellule transformée par un polynucléotide selon une quelconque des revendications 2 à 4.

8. Procédé de criblage de produits fongicides, **caractérisé en ce qu'**il comprend la mesure de l'activité kinase d'une CAK non cycline-dépendante selon une quelconque des revendications 1 ou 5, en présence de chacun des produits dont on souhaite déterminer les propriétés fongicides, et la sélection des produits ayant un effet inhibiteur sur cette activité.

## Claims

1. Protein-kinase possessing a non-cyclin dependent CAK activity, **characterised in that** it complies with the sequence shown in the appended list of sequences under the number SEQ ID No: 2.

2. Polynucleotide coding for a protein-kinase according to claim 1.

3. Polynucleotide according to claim 2, **characterised in that** it complies with the sequence shown in the appended list of sequences under the number SEQ ID No: 1.

4. Polynucleotide coding for a protein-kinase possessing the following characteristics:
- it does not comprise the unit GxGx(Y/F)GxV, in which G represents glycine, x represents any amino acid, Y/F represents either tyrosine or phenylalanine, and V represents valine;
- it has a non-cyclin dependent CAK activity;
- said polynucleotide being **characterised in that** it is able to be isolated from *Candida albicans* by means of a nucleic acid probe consisting of a fragment of at least 18 bp of the polynucleotide SED ID No: 1 coding for a specific peptide sequence of a protein-kinase according to claim 1, or of the complementary thereof.

5. Protein-kinase coded by a nucleic acid according to claim 4.

6. Recombinant vector resulting from the insertion of a polynucleotide according to any one of claims 2 to 4 in an appropriate vector.

7. Cell transformed by a polynucleotide according to any one of claims 2 to 4.

8. Method of screening fungicidal products, **characterised in that** it comprises the measurement of the kinase activity of a non-cyclin dependent CAK according to either one of claims 1 or 5 in the presence of each of the products whose fungicidal properties it is wished to determine, and the selection of the products that have an inhibiting effect on this activity.

## Patentansprüche

1. Proteinkinase, die eine nicht-Cyclin-abhängige CAK-Aktivität besitzt, **dadurch gekennzeichnet, dass** sie der Sequenz entspricht, die im beigefügten Sequenzprotokoll unter der Nummer SEQ ID NO: 2 dargestellt ist.

2. Polynucleotid, das für eine Proteinkinase nach Anspruch 1 codiert.

3. Polynucleotid nach Anspruch 2, **dadurch gekennzeichnet, dass** es der Sequenz entspricht, die im beigefügten Sequenzprotokoll unter der Nummer SEQ ID NO: 1 dargestellt ist.

4. Polynucleotid, das für eine Proteinkinase codiert, die die folgenden Charakteristika besitzt:
- sie ist frei von dem Motiv GxGx(Y/F)GxV, worin G Glycin darstellt, x eine beliebige Aminosäure darstellt, Y/F Tyrosin oder Phenylalanin darstellt, V Valin darstellt;
- sie besitzt eine nicht-Cyclin-abhängige CAK-Aktivität;
wobei das Polynucleotid **dadurch gekennzeichnet ist, dass** es ausgehend von *Candida albicans* mit Hilfe einer Nucleinsäuresonde isoliert werden kann, die durch ein Fragment mit wenigstens 18 bp des Polynucleotids SEQ ID NO: 1, das für eine spezifische Peptidsequenz einer Proteinkinase nach Anspruch 1 codiert, oder durch seine komplementäre Sequenz gebildet wird.

5. Proteinkinase, die durch eine Nucleinsäure nach Anspruch 4 codiert wird.

6. Rekombinanter Vektor, der aus der Insertion eines Polynucleotids nach einem der Ansprüche 2 bis 4 in einen geeigneten Vektor resultiert.

7. Zelle, die durch ein Polynucleotid nach einem der Ansprüche 2 bis 4 transformiert ist.

8. Verfahren zur Durchmusterung von fungiziden Produkten, **dadurch gekennzeichnet, dass** es Messen der Kinaseaktivität einer nicht-Cyclin-abhängigen CAK nach Anspruch 1 oder 5 in Gegenwart jedes der Produkte, von dem man die fungiziden Eigenschaften bestimmen möchte, und die Selektion der Produkte, die eine inhibitorische Wirkung auf diese Aktivität haben, umfasst.
